# EUROPEAN PATENT SPECIFICATION

(11) **EP 2 556 040 B1**
(45) Date of publication and mention of the grant of the patent: **27.12.2017**
(21) Application number: 11718776.5
(22) Date of filing: 07.04.2011
(51) Int. Cl.: C07B 57/00, C07C 227/34

(54) **INDUSTRIAL PROCESS FOR THE SYNTHESIS OF (S)-(+)-PREGABALIN**
INDUSTRIEVERFAHREN FÜR DIE SYNTHESE VON (S)-PREGABALIN
PROCÉDÉ INDUSTRIEL DE SYNTHÈSE DE (S)-(+)-PRÉGABALINE

(30) Priority: 08.04.2010 HU P1000186
(43) Date of publication of application: 13.02.2013
(73) Proprietor: Richter Gedeon Nyrt., 1103 Budapest (HU)
(72) Inventor: NEU, József, H-1133 Budapest (HU); FOGASSY, Elemér, H-2030 Érd (HU); SZALMA, Nikoletta, H-2500 Esztergom (HU); KÁLVIN, Péter, H-2509 Esztergom-Kertváros (HU); SCHINDLER, József, H-2151 Fót (HU); JAKAB, Gábor, H-2510 Dorog (HU); GARADNAY, Sándor, H-2500 Esztergom (HU); PÁLOVICS, Emese, H-1081 Budapest (HU)
(74) Representative: Hoffmann Eitle
(86) International application number: PCT/HU2011/000030
(87) International publication number: WO 2011/124934

(56) References cited:
- WO-A1-96/40617

## Description

The invention relates to an industrial process for the synthesis of (*S*)-(+)-pregabalin the chemical name of which is (*S*)-(+)-3-(amino-methyl)-5-methyl-hexanoic acid in high purity.

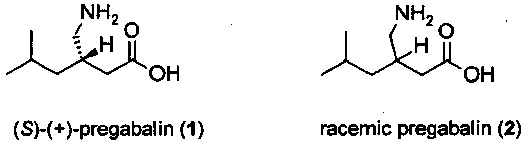

The synthesis of (*S*)-(+)-pregabalin (1) is described in US 5637767 Patent. The racemic pregabalin is crystallized with (*S*)-(+)-mandelic acid from isopropanol containing 3% of water which was decomposed in tetrahydrofuran (THF) and the obtained crude (*S*)-(+)-pregabalin was recrystallized from isopropanol again. The stoichiometry of the used racemic pregabalin (2) : (*S*)-(+)-mandelic acid : isopropanol: THF = 3.63 : 5.43 : 51.0 : 16.3.

WO 2009 122215 A1 describes the synthesis of (*S*)-(+)-pregabalin (1) as well. According to the description (2R,3R)-(+)- Tartaric acid was used in the mixture of n-butanol : water = 9 : 1. The crude diastereomeric salt was recrystallized from the mixture of isopropanol : water = 4 : 1 and decomposed with *N*,*N*-diisopropil-amine. The crude (*S*)-(+)-pregabalin (1) was recrystallized from the mixture of isopropanol : water = 3 : 1. The disadvantage of this process is the big excess of the starting materials.

The invention relates to a process for the synthesis of (*S*)-(+)-pregabalin (1) starting from racemic pregabalin (2) in aqueous solution. The aqueous racemic pregabalin (2) was reacted with a chiral α-hydroxy-carboxylic acid or with the mixture of α-hydroxy-carboxylic acids in desired case in the presence of an achiral acid and then the obtained diastereomeric salt is deliberated. In desired case the residue racemic pregabalin (2) is regenerated.
As the α-hydroxy-carboxylic acid, (2R,3R)-(+)- Tartaric acid or (*S*)-(+)-mandelic acid is used.

The racemic pregabalin (2) is reacted with 0.5-1.0 mole equivalent of chiral α-hydroxy-carboxylic acid and 0.5-0.0 mole equivalent of other chiral α-hydroxy-carboxylic acid in desired case in the presence of 0.5-0.0 mole equivalent of achiral acid.

According to another embodiment of the process of the invention the racemic pregabalin (2) is reacted in aqueous solution with 0.5-0.9 mole equivalent of chiral α-hydroxy-carboxylic acid and 0.5-0.1 mole equivalent of other chiral α-hydroxy-carboxylic acid and in desired case in the presence of 0.1-0.5 mole equivalent of achiral acid.

The obtained salt of (*S*)-(+)-pregabalin (1) with chiral acid is preferably filtered and then recrystallized from water and reacted with aqueous ammonium hydroxide. The crystalline (*S*)-(+)-pregabalin (1) is filtered, recrystallized from water.

The salt of (*S*)-(+)-pregabalin (1) with chiral α-hydroxy-carboxylic acid is filtered preferably after 1 hour crystallization and recrystallized from water.

The salt of (*S*)-(+)-pregabalin (1) with chiral α-hydroxy-carboxylic acid is filtered after 12 hours crystallization and recrystallized twice from water.

The mother liquid was crystallized for 12 hours, it was filtered, the obtained crude diastereomeric salt and the mother liquid of the previous fraction was unified. Chiral α-hydroxy-carboxylic acid and achiral acid was added to the mixture of racemic pregabalin (2) and the obtained (*S*)-(+)-pregabalin (1) diastereomeric salt was crystallized.

The crude diastereomeric salt containing mother liquids were worked up on the same way.

As chiral α-hydroxy-carboxylic acid (2R,3R)-(+)- Tartaric acid or (*S*)-(+)- mandelic acid was used.

As achiral acid preferably hydrochloric acid, benzoic acid, salicylic acid or phenyl acetic acid was used.

The recrystallized (*S*)-(+)-pregabalin (1) containing diastereomeric salt was heated with aqueous ammonium hydroxide until the dissolution was completed and then the crystalline (*S*)-(+)-pregabalin (1) is filtered.

In desired case the (*S*)-(+)-pregabalin (1) is recrystallized.

The obtained mother liquid is acidified with hydrochloric acid and the crystalline (*S*)-(+)-mandelic acid is filtered.

The first mother liquid of the crystallization is reacted with ammonium hydroxide, the mixture of (*R*)-(-)-pregabalin was filtered and in desired case this process is repeated.

The racemic and the mixture of (*R*)-(-)-pregabalin is reacted with oxalic acid which is equivalent with the racemic part and with hycrochloric acid which is equivalent with the enantiomer. The racemic pregabalin oxalate is reacted with aqueous ammonium hydroxide and the precipitated racemic pregabalin (2) is filtered.

Ammonium acetate was solved in the mother liquid obtained by the resolution with *(R,R)-(+)-* tartaric acid. Then the racemic pregabalin (2) was precipitated with ammonium hydrogen tartarate. The racemic pregabalin (2) can be regenerated and given back to the resolution process.

The object of the invention was the elaboration of a new, industrially applicable process according to which the preparation of the (*S*)-(+)-pregabalin (1) is cost effective, can be carried out easily with simple technology.

In accordance with the present invention it has been found that the α-hydroxyl carboxylic acids such as tartaric acid, and mandelic acid are suitable for the preparation of (*S*)-(+)-pregabalin (1) only in aqueous solution starting from the racemic form thereof.

Surprisingly it was found that good enantiomeric selection can be made with 0.5 - 0.9 mole equvivalent ratio of these resolution agents calculated to the racemic pregabalin (2). The enantiomeric selection can be improved if the other mole equivalent amount of the required acid is substituted by another chiral or achiral acid and as resolution agent a mixture of acids is used.

These mixture of acids resulted good diastereomeric selectivity when aqueous alcohol was used as solvent.

The mixture of (*S*)-(+)-mandelic acid and (*R,R*)-(+)- tartaric acid, hydrochloric acid, benzoic acid, salicilic acid, phenyl acetic acid was used in the mole ratio of 1:1.

It was surprisingly found that by the recrystallization of the crude diastereomer product the aqueous solution preferably contains another chiral or achiral acid which is different from the diastereomer.

It has been found that at the diastereomeric crystallization from the racemic compound and the solution of the resolution mixture acids kinetic control occurs and therefore (*S*)-(+)-pregabalin (1) containing diastereomeric salt can be precipitated easier.

The reaction mixture was filtered rapidly after the crystallization has begun. This pure diastereomer product can be obtained only with the recrystallization of the long (12 hours) crystallizing diastereomer.

It has also been found that in case of using the kinetic control the less pure diastereomer product crystallized for a long period (12 hours) can be used in the next resolution again.

It has been found that the enantiomer can be deliberated by aqueous ammonium hydroxide form the (*S*)-(+)-pregabalin (1) containing diastereomeric salt.

The main amount of the resolution agent e. g. (*S*)-(+)-mandelic acid is precipitated during the separation of (*S*)-(+)-pregabalin (1) with aqueous ammonium hydroxide. The obtained mother liquid is acidified and the precipitated resolution agent can be regenerated this way.

It has been found that treating the mother liquid obtained by the filtration of the crude diastereomeric salt with ammonium hydroxide the mixture of pregabalin enantiomers is precipitated which contains racemic and (*R*)-(-)-pregabalin.

It was surprisingly found that in aqueous solution the mixture of enantiomers containing racemic pregabalin (2) was reacted with mole equivalent of oxalic acid and the obtained product is precipitated. The separation was carried out preferably with mixture of acids, e.g. the mixture of oxalic acid and hydrochloric acid.

It follows from the foregoing that the main part of the non resolvated pregabalin can be deliberated from racemic pregabalin oxalate with aqueous ammonium hydroxide.

It has also been found that if ammonium acetate is added to the mother liquid of the resolution with (2R,3R)-(+)- Tartaric acid then in addition to ammonium hydrogen tartarate surprisingly the racemic pregabalin (2) of the solution is also precipitated.

If this mixture is treated with ammonium hydroxide then the racemic pregabalin (2) can be regenerated and given back to the resolution process.

The process is illustrated by the following examples:

### Example 1

In a flask 1.6 g (0.01 mole) of racemic pregabalin (2), 0.8 g (0.0053 mole) of (*S*)-(+)-mandelic acid and 0.42 cm³ (0.0051 mole) of 37% hydrochloric acid diluted with 3 cm³ of water was added. The reaction mixture was heated to 70°C, seeded, stirred for 10 minutes and the obtained diastereomer was washed with 2 x 0,4 cm³ of water.

1.0 g (66.7%) of (*S*)-(+)-pregabalin (1) containing diastereomer salt is obtained, [α]_{D}=+10.7(c=1, water)

### Example 2

In a flask 5.0 g (0.0314 mole) of racemic pregabalin (2), 4.31 g (0.0283 mole) of (*S*)-(+)-mandelic acid, 1.55 cm³ 6N (0.0031 mole) of hydrochloric acid and 36 cm³ of water was added. The reaction mixture was heated to 60-65°C, the obtained solution was cooled and seeded at 40-45°C. Then the reaction mixture was cooled to 0-5°C, stirred for 2 hours at this temperature and filtered. The obtained diastereomer was washed with 2x5 cm³ of isopropanol (0-5°C).

3.64 g (74.4%) of (*S*)-(+)-pregabalin (1) containing diastereomer salt is obtained, [α]_{D}=+11.8 (c=1, water)

### Example 3

In a flask 9.54 g (0.06 mole) of racemic pregabalin (2), 4.50 g (0.03 mole) of (R,R)-(+)- tartaric acid, 15.0 ml 2N of hydrochloric acid and 12 cm³ of water was added. The reaction mixture was heated to 58-62°C then cooled to 40-42°C and seeded. Then the reaction mixture was cooled to 0-5°C, stirred for 2 hours at this temperature and filtered. The obtained diastereomer was washed with 2x5 cm³ of isopropanol (0-5°C).

5.3 g (57%) of (*S*)-(+)-pregabalin (1) containing diastereomer salt is obtained, [α]_{D}=+11.9 (c=1, water)

### Reference example 4

In a flask 1.6 g (0.01 mole) of racemic pregabalin (2), 1.2 g (0.01 mole) of benzoic acid and 1.6 g (0.011 mole) of (*S*)-(+)-mandelic acid was added to the mixture of 12,8 cm³ of isopropanol and 0,4 cm³ of water. The reaction mixture was heated to 50 °C, seeded and the obtained crystalline diastereomer was filtered.

1.32 g (79.3%) of (*S*)-(+)-pregabalin (1) containing diastereomer salt is obtained, [α]_{D}=+11.8 (c=1, water)

### Reference example 5

In a flask 1.6 g (0.01 mole) of racemic pregabalin (2), 1.4 g (0.01 mole) of salicylic acid and 1.6 g (0.011 mole) of (*S*)-(+)-mandelic acid was added to the mixture of 12.8 cm³ of isopropanol and 0,4 cm³ of water. The reaction mixture was heated to 50 °C, seeded and the obtained crystalline diastereomer was filtered.

1.08 g (64.9%) of (*S*)-(+)-pregabalin (1) containing diastereomer salt is obtained, [α]_{D}=+11.5 (c=1, water)

### Reference example 6

In a flask 1.6 g (0.01 mole) of racemic pregabalin (2), 1.4 g (0.01 mole) of phenyl acetic acid and 1.6 g (0.011 mole) of (*S*)-(+)-mandelic acid was added to the mixture of 12.8 cm³ of isopropanol and 0.4 cm³ of water. The reaction mixture was heated to 50 °C, seeded and the obtained crystalline diastereomer was filtered.

1.2 g (72%) of (*S*)-(+)-pregabalin (1) containing diastereomer salt is obtained, [α]_{D}=+12.1 (c=1, water)

### Example 7

In a flask 1.6 g (0.01 mole) of racemic pregabalin (2), 0.8 g (0.0053 mole) of (*S*)-(+)-mandelic acid and 0.8 g (0.0053 mole) of (2R,3R)-(+)- Tartaric acid was solved in 3 cm³ of water. The reaction mixture was heated to 60 °C, seeded and the obtained crystalline product was stirred for 40 minutes, then filtered and washed with 2 x 0.4 cm³ of water.

1.08 g (69%) of (*S*)-(+)-pregabalin (1) containing diastereomer salt is obtained, [α]_{D}= +12.0 (c=1, water)

### Example 8

In a flask 6.36 g (0.04 mole) of racemic pregabalin (2), 1.22 g (0.008 mole) of (*S*)-(+)-mandelic acid and 4.8 g (0.032 mole) of (2R,3R)-(+)- Tartaric acid was solved in 20 cm³ of water. The reaction mixture was heated to 60 °C, seeded and the obtained crystalline product was stirred for 40 minutes at room temperature and for 1 hour at 0-5°C, then filtered and washed with 2x3 cm³ of water.

4.17 g (67%) of (*S*)-(+)-pregabalin (1) containing diastereomer salt is obtained, [α]_{D}=+11.8 (c=1, water)

### Example 9

In a flask 6.36 g (0.04 mole) of racemic pregabalin (2), 1.83 g (0.012 mole) of (*S*)-(+)-mandelic acid and 4.2 g (0.028 mole) of (2R,3R)-(+)- Tartaric acid was solved in 20 cm³ of water. The reaction mixture was heated to 60 °C, seeded and the obtained crystalline product was stirred for 40 minutes at room temperature and for 1 hour at 0-5°C, then filtered and washed with 2x3 cm³ of water.

3.82 g (61%) of (*S*)-(+)-pregabalin (1) containing diastereomer salt is obtained, [α]_{D}=+11.3 (c=1, water)

### Example 10

In a flask 16 g (0.1 mole) of racemic pregabalin (2), 8 g (0.053 mole) of (*S*)-(+)-mandelic acid was solved in 4.2 cm³ (0.051 mole) of hydrochloric acid and 30 cm³ of water. The reaction mixture was heated to 70 °C, seeded, the obtained crystalline product was filtered in 20 minutes and washed with 2x4 cm³ of water.

9.14 g (58.8%) of (*S*)-(+)-pregabalin (1) containing diastereomer salt is obtained, [α]_{D}= +11.4 (c=1, water)

The mother liquid was stirred for further 12 hours and the obtained crystalline product was filtered.

2.8 g (18%) of further (*S*)-(+)-pregabalin (1) containing diastereomer salt is obtained, [α]_{D}=+9.2 (c=1, water)

### Example 11

In a flask 2.8 g (0.009 mole) of diastereomer salt obtained in Example 10 was solved in 0.146 g (0.004 mole) of hydrochloric acid and 5.3 cm³ of water. The reaction mixture was heated to 70 °C, seeded, the obtained crystalline product was filtered in 15 minutes and washed with 2x0.2 cm³ of water.

1.7 g (60.7%) of (*S*)-(+)-pregabalin (1) containing diastereomer salt is obtained, [α]_{D}=+11.6 (c=1, water)

### Example 12

In a flask 2.8 g (0.009 mole) of second, crude diastereomer salt fraction ([α]_{D} = +9.2 (c=1, water), obtained in Example 10, 13.2 g of racemic pregabalin (2) 6.6 g (0.0434 mole) of (*S*)-(+)-mandelic acid was solved in 3.5 cm³ (0.0422 mole) of 37% hydrochloric acid and 30 cm³ of water. The reaction mixture was heated to 50 °C, seeded, the obtained crystalline product was filtered in 15 minutes and washed with 2x4 cm³ of water.

9.1 g (58.5) of (*S*)-(+)-pregabalin (1) containing diastereomer salt is obtained, [α]_{D}= +11.5 (c=1, water)

### Example 13

In a flask 8.35 g (0.0268 mole) of (*S*)-(+)-pregabalin*(*S*)-(+)-mandelic acid diastereomer salt obtained in Example 10 ([α]_{D} = +11.4 (c=1, water) is added. 0.4 g (0.0026 mole) (*S*)-(+)-mandelic acid was solved in 20 cm³ of water. The reaction mixture was seeded, the obtained crystalline product was filtered in 15 minutes.

6.74 g (80.7%) of (*S*)-(+)-pregabalin (1) containing diastereomer salt is obtained, [α]_{D}=+11.8 (c=1, water)

### Example 14

In a flask 14 g (0.088 mole) of racemic pregabalin (2), 6 g (0.0395 mole) of (*S*)-(+)-mandelic acid was solved in 4.2 cm³ (0.051 mole) of hydrochloric acid and 25 cm³ of water.

The mother liquid of Example 13 and the crude 2.32 g (0.0075 mole) of (*S*)-(+)-pregabalin*(*S*)-(+)-mandelic acid salt ([α]_{D} = +9.2 (c=1, water)) obtained in Example 10 was added.

The reaction mixture was heated to 70 °C and seeded. Then the crystalline product was filtered in 15 minutes. The obtained product was washed with 2x4 cm³ of water.

8.9 g (57.4%) of (*S*)-(+)-pregabalin (1) containing diastereomer salt is obtained, [α]_{D}=+11.3 (c=1, water).

### Example 15

In a flask 16 g (0.1 mole) of racemic pregabalin (2), 8 g (0.053 mole) of (*S*)-(+)-mandelic acid was solved in 4.2 cm³ (0.051 mole) of 37% hydrochloric acid and 25 cm³ of water.

The reaction mixture was heated to 70-80 °C, than cooled to room temperature and seeded. The crystalline product was filtered in 15-20 minutes during stirring. Then the reaction mixture was kept at room temperature for 10-12 hours and the obtained product was filtered. The diastereomer salt was washed with 2x4 cm³ of water.

12.4 g (79.24%) of (*S*)-(+)-pregabalin (1) containing diastereomer salt (I. salt) is obtained, [α]_{D}=+10.9 (c=1, water).

The obtained crude I diastereomer salt was solved in 20 cm³ of water, 0.4 g of (*S*)-(+)-mandelic acid was added. The reaction mixture was heated to 70-80°C, and then at room tempereature was seeded. The precipitated product was kept at room temperature for 2 hours, the obtained product was filtered and washed with 2x4 cm³ of water.

11.9 g (76%) of (S)-(+)-pregabalin (1) containing diastereomer II. salt is obtained, [α]_{D} = +12.4 (c=1, water).

The obtained diastereomer II. salt was recrystallized from 15 cm³ of water.

9.2 g (58.8%) of (S)-(+)-pregabalin*-mandelic acid salt diastereomer salt III.is obtained, [α]_{D} = +12.2 (c=1, water).

### Example 16

The mother liquids of II. and III. salts were unified, 14.4 g (0.091 mole) of racemic pregabalin (2), 6.9 g (0.045 mole) of (*S*)-(+)-mandelic acid and then 3,6 cm³ (0,044 mole) of 37% hydrochloric acid was added.

The reaction was carried out according to Example 15.

12.1 g (77.8%) of (S)-(+)-pregabalin*-mandelic acid diastereomer salt I: is obtained, [α]_{D}= +11.3 (c=1, water).

### Example 17

In a flask 36 g (0.226 mole) of racemic pregabalin (2), 35 g (0.233 mole) of (2R,3R)-(+)- Tartaric acid was solved in 180 cm³ of water at 48-52°C. The reaction mixture was cooled to 20-22°C and seeded. Then it was cooled to 5°C in 30 minutes and it was stirred at temperature 0-5°C for 2 hours and filtered. The obtained diastereomer product was washed with 2x35 cm³ aceton (0-5 °C).

24.7 g (70.4%) of (*S*)-(+)-pregabalin (1) containing diastereomer salt is obtained, [α]_{D}=+11.5 (c=1, water)

### Example 18

In a flask 21.63 g (0.07 mole) of (*S*)-(+)-pregabalin* (2R,3R)-(+)- Tartaric acid diastereomer salt obtained in Example 17 and 1.05 g (0.0007 mole) of (*S*)-(+)-mandelic acid was solved in the mixture of 60.5 cm³ of isopropanol and 2.5 cm³ of water.

The solution was cooled to 20-25°C in 30 minutes. Then it was cooled further to 0-5°C in 30 minutes, stirred for 1 hour, filtered and washed with 2x0.2 cm³ of isopropanol.

19.5 g (90.2%) of (*S*)-(+)-pregabalin (1) containing diastereomer salt is obtained, [α]_{D}= +12 (c=1, water)

### Example 19

In a flask 18 g (0.058 mole) of (*S*)-(+)-pregabalin* (2R,3R)-(+)- Tartaric acid diastereomer salt obtained in Example 18, 24 cm³ of water and 13 cm³ of 25% ammonium hydroxide was added.

The solution was heated to 60-65°C, then cooled to 0-5°C, stirred for 1 hour, filtered and washed with the mixture of 2 x 3.5 cm³ of water and 3.5 cm³ of 25% ammonium hydroxide. 7.51 g (81.1%) of (*S*)-(+)-pregabalin (1) is obtained, [α]_{D}= +11.9 (c=1, water).

### Example 20

In a flask 13.36 g (0.058 mole) of (*S*)-(+)-pregabalin* (2R,3R)-(+)- Tartaric acid diastereomer salt obtained in Example 18, 32 cm³ of water and 7.2 cm³ of 25% ammonium hydroxide was added.

The solution was heated to 60-65°C, then cooled to 0-5°C, stirred for 1 hour, filtered and washed with the mixture of 2 x 7 cm³ of water.

4.65 g (81.1%) of (*S*)-(+)-pregabalin (1) is obtained, [α]_{D}= +12.2 (c=1, water).

The obtained mother liquid was stirred for 1 hour, the precipitated product was filtered and 0.28 g of (*S*)-(+)-pregabalin (1) is obtained, [α]_{D}= +0.2 (c=1, water).

### Example 21

In a flask 7.3 g (0.046 mole) of (*S*)-(+)-pregabalin (1), 43.8 cm³ of isopropanol and 29.2 cm³ of water was added. The reaction mixture was heated to reflux and filtered. The solution was cooled to 0-5°C, filtered and washed with 2x7 cm³ of isopropanol.

6.05 g (82.9%) of (*S*)-(+)-pregabalin (1) is obtained, ee:100%.

### Example 22

In a flask 18.4 g (0.059 mole) of (*S*)-(+)-pregabalin*(*R*,*R*)-(+)-mandelic acid diastereomer salt (III) obtained in Example 15 or 16 was added.

6.1 g of (*S*)-(+)-pregabalin*(*R*,*R*)-(+)-mandelic acid diastereomer salt (III) was solved in 10 cm³ of water and 10 cm³ of cc. ammonium hydroxide at reflux temperature.

The reaction mixture was cooled to 20-22°C, the precipitated product was stirred for 2 hours, filtered and washed with 3 x 1 cm³ of water and dried.

2.2 g of (*S*)-(+)-pregabalin (1) is obtained, [α]_{D}= +11.5 (c=1, water).

Further 6.1 g of (*S*)-(+)-pregabalin*(*R*,*R*)-(+)-mandelic acid diastereomer salt (III) and 3 cm³ of cc. ammonium hydroxide was added to the mother liquid. The reaction mixture was heated to reflux temperature.

Then the reaction was carried out according to the previous steps.

3 g of (*S*)-(+)-pregabalin (1) is obtained, [α]_{D}= +11.5 (c=1, water).

Further 6.1 g of (*S*)-(+)-pregabalin*(*R*,*R*)-(+)-mandelic acid diastereomer salt (III) and 3 cm³ of cc. ammonium hydroxide was added to the mother liquid. The reaction mixture was heated to reflux temperature.

Then the reaction was carried out according to the previous steps.

2.8 g of (*S*)-(+)-pregabalin (1) is obtained, [α]_{D}= +10.9 (c=1, water).

8 g (85.5%) of (*S*)-(+)-pregabalin (1) is obtained from 18.4 g of (*S*)-(+)-pregabalin*(*R*,*R*)-(+)-mandelic acid diastereomer salt (III).

### Example 23

The mother liquid according to the Example 22 contains 8.9 g of (*S*)-(+)-mandelic acid. It was acidified with 20 cm³ of cc (37%) hydrochloric acid and the precipitated crystals were kept for 2 hours at room temperature, then filtered, washed with 2x3 of 10% hydrochloric acid and dried.

6.1 g (68.2%) of regenerated mandelic acid is obtained, [α]_{D}= +151.4 (c=1, water).

### Example 24

The reaction was carried out according to Example 15. The mother liquid of diastereomer salt I was added to 8 cm³ of ammonium hydroxide and was kept for 2 hours at room temperature. The precipitated 8.5 g of enantiomer mixture which contains about 4 g of (R)-(-)-pregabalin excess was filtered and dried.

8.5 g of enantiomer mixture and 2.6 g of oxalic acid was solved in 1.1 cm³ of cc (37%) hydrochloric acid and 18 cm³ of water at 80 °C. The obtained solution was kept at room temperature for 30 minutes and filtered.

The oxalate salt was washed with 2x4 cm³ of water and then it was solved in warm mixture of 20 cm³ of water and 10 cm³ of ammonium hydroxide. It was kept at room temperature for 45 minutes and filtered (washed with 2 cm³ of water).

3.52 g of regenerated racemic pregabalin (2) is obtained, [α]_{D}= 0.

### Example 25

The mother liquid obtained according to Example 17 was evaporated to 190 g and then 15 g of ammonium acetate was added. The reaction mixture was heated to reflux temperature and cooled. It was stirred for 1 hour at ice cooling, filtered, washed with 10 cm³ of water and dried.

9.23 g (83%) of racemic pregabalin (2) is obtained, [α]_{D}= 0.2 (c=1, water).

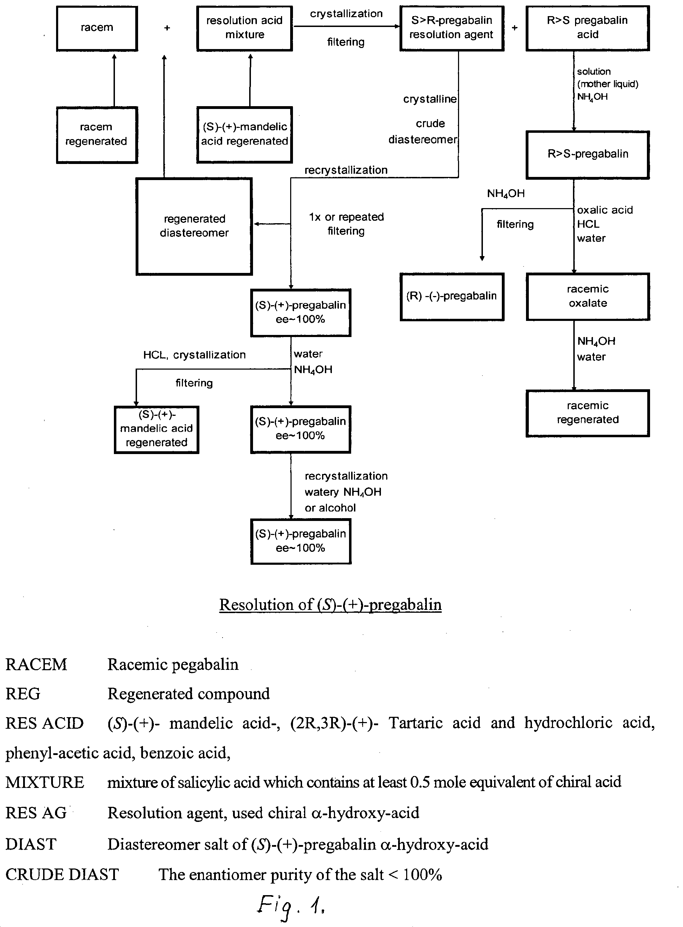

## Claims

1. Process for the preparation of (S)-(+)-pregabalin (1), **characterized in that** racemic pregabalin (2) is dissolved in water,
either reacted with a chiral α-hydroxy-carboxylic acid in the presence of an achiral acid, or reacted with a mixture of chiral α-hydroxy-carboxylic acids optionally in the presence of an achiral acid, wherein the chiral α-hydroxy-carboxylic acid is (2R,3R)-(+)- Tartaric acid or (S)-(+)-mandelic acid,
(S)-(+)-pregabalin (1) is liberated from the obtained diastereomer salt and
optionally, the residue of racemic pregabalin (2) is regenerated.

2. Process according to claim 1, **characterized in that** racemic pregabalin (2) is reacted in aqueous solution with 0.5-1.0 mole equivalent of a first chiral α-hydroxy-carboxylic acid and 0.5-0.0 mole equivalent of a second chiral α-hydroxy-carboxylic acid in the presence of an achiral acid.

3. Process according to claim 1, **characterized in that** racemic pregabalin (2) is reacted with 0.5-0.9 mole equivalent of a first chiral α-hydroxy-carboxylic acid and 0.5-0.1 mole equivalent of a second chiral α-hydroxy-carboxylic acid in the presence of achiral acid in aqueous solution.

4. Process according to any claims of 1-3, **characterized in that** the (S)-(+)-pregabalin (1) chiral α-hydroxy-carboxylic acid salt is filtered, crystallized from water and reacted by aqueous ammonium hydroxide, and the obtained crystalline (S)-(+)-pregabalin (1) product is filtered and recrystallized from water.

5. Process according to any claims of 1-4, **characterized in that** the (S)-(+)-pregabalin (1) chiral α-hydroxy-carboxylic acid salt is crystallized for 1 hour, filtered and recrystallized from water.

6. Process according to any claims of 1-4 **characterized in that** the (S)-(+)-pregabalin (1) chiral α-hydroxy-carboxylic acid salt is crystallized for 12 hours, filtered and recrystallized twice from water.

7. Process according to claim 5 **characterized in that** the mother liquid is crystallized for 12 hours, filtered and the obtained diastereomer salt and the mother liquid are unified, racemic pregabalin (2) and chiral α-hydroxy-carboxylic acid are added and then the (S)-(+)-pregabalin (1) containing diastereomer salt is crystallized.

8. Process according to any claims of 1-7 **characterized in that** as achiral acid hydrochloric acid, benzoic acid, salicylic acid or phenyl-acetic acid is used.

9. Process according to any claims of 1-8 **characterized in that** the recrystallized (S)- (+)-pregabalin (1) containing diastereomer salt is solved in warm aqueous ammonium hydroxide and the precipitated (S)-(+)-pregabalin (1) is filtered.

10. Process according to any claims of 1-9 **characterized in that** (S)-(+)-pregabalin (1) is recrystallized.

11. Process according to claim 9, **characterized in that** the obtained mother liquid is acidified with hydrochloric acid and the precipitated (S)-(+)-mandelic acid is filtered and, optionally, this process is repeated.

12. Process according to claim 4, **characterized in that** the first mother liquid is reacted with ammonium hydroxide and the precipitated mixture of racemic and (R)-(-)-pregabalin is filtered.

13. Process according to claims 3 or 12, **characterized in that** the obtained mixture of racemic and (R)-(-)-pregabalin is reacted with oxalic acid which is mole equivalent of the racemic amount and hydrochloric acid which is mole equivalent of the enantiomer product, then the precipitated racemic pregabalin (2), is filtered.

14. Process according to claims 1 or 2, **characterized in that** ammonium acetate is dissolved in the mother liquid obtained by the resolution with (2R,3R)-(+)-Tartaric acid and then ammonium-hydrogen tartarate and racemic pregabalin (2) are precipitated.

## Patentansprüche

1. Verfahren zur Herstellung von (S)-(+)-Pregabalin (1), **dadurch gekennzeichnet, dass** racemisches Pregabalin (2) in Wasser gelöst wird,
entweder mit einer chiralen α-Hydroxycarbonsäure in Gegenwart einer achiralen Säure umgesetzt wird oder mit einer Mischung von chiralen α-Hydroxycarbonsäuren, gegebenenfalls in Gegenwart einer achiralen Säure, umgesetzt wird, wobei die chirale α-Hydroxycarbonsäure (2R, 3R)-(+)-Weinsäure oder (S)-(+)-Mandelsäure ist,
(S)-(+)-Pregabalin (1) von dem erhaltenen Diastereomerensalz befreit wird und
der racemische Pregabalin (2)-Rückstand gegebenenfalls regeneriert wird.

2. Verfahren gemäss Anspruch 1, **dadurch gekennzeichnet, dass** racemisches Pregabalin (2) in einer wässrigen Lösung mit 0,5 bis 1,0 Moläquivalenten einer ersten chiralen α-Hydroxycarbonsäure und 0,5 bis 0,0 Moläquivalenten einer zweiten chiralen α-Hydroxycarbonsäure in Gegenwart einer achiralen Säure umgesetzt wird.

3. Verfahren gemäss Anspruch 1, **dadurch gekennzeichnet, dass** racemisches Pregabalin (2) mit 0,5 bis 0,9 Moläquivalenten einer ersten chiralen α-Hydroxycarbonsäure und 0,5 bis 0,1 Moläquivalenten einer zweiten chiralen α-Hydroxycarbonsäure in Gegenwart einer achiralen Säure in einer wässrigen Lösung umgesetzt wird.

4. Verfahren gemäss einem der Ansprüche 1 bis 3, **dadurch gekennzeichnet, dass** das chirale (S)-(+)-Pregabalin (1)-α-Hydroxycärbonsäuresälz filtriert, aus Wasser kristallisiert und mit wässrigem Ammoniumhydroxid umgesetzt wird und das erhaltene krystalline (S)-(+)-Pregabalin (1)-Produkt filtriert und aus Wasser umkristallisiert wird.

5. Verfahren gemäss einem der Ansprüche 1 bis 4, **dadurch gekennzeichnet, dass** das chirale (S)-(+)-Pregabalin (1)-α-Hydroxycärbonsäuresälz für 1 Stunde kristallisiert, filtriert und aus Wasser umkristallisiert wird.

6. Verfahren gemäss einem der Ansprüche 1 bis 4, **dadurch gekennzeichnet, dass** das chirale (S)-(+)-Pregabalin (1)-α-Hydroxycärbonsäuresälz für 12 Stunden kristallisiert, filtriert und zweimal aus Wasser umkristallisiert wird.

7. Verfahren gemäss Anspruch 5, **dadurch gekennzeichnet, dass** die Mutterlauge für 12 Stunden kristallisiert, filtriert und das erhaltene Diastereomerensalz und die Mutterlauge vereinigt werden, racemisches Pregabalin (2) und chirale α-Hydroxycarbonsäure zugegeben werden und dann das (S)-(+)-Pregabalin (1)-haltige Diastereomerensalz kristallisiert wird.

8. Verfahren gemäss einem der Ansprüche 1 bis 7, **dadurch gekennzeichnet, dass** als achirale Säure Salzsäure, Benzoesäure, Salicylsäure oder Phenylessigsäure verwendet wird.

9. Verfahren gemäss einem der Ansprüche 1 bis 8, **dadurch gekennzeichnet, dass** das umkristallisierte (S)-(+)-Pregabalin (1)-haltige Diastereomerensalz in warmem wässrigen Ammoniumhydroxid gelöst wird und das ausgefällte (S)-(+)-Pregabalin (1) filtriert wird.

10. Verfahren gemäss einem der Ansprüche 1 bis 9, **dadurch gekennzeichnet, dass** (S)-(+)-Pregabalin (1) umkristallisiert wird.

11. Verfahren gemäss Anspruch 9, **dadurch gekennzeichnet, dass** die erhaltene Mutterlauge mit Salzsäure angesäuert und die ausgefällte (S)-(+)-Mandelsäure filtriert wird und dieses Verfahren gegebenenfalls wiederholt wird.

12. Verfahren gemäss Anspruch 4, **dadurch gekennzeichnet, dass** die erste Mutterlauge mit Ammoniumhydroxid umgesetzt und die ausgefällte Mischung aus racemischem und (R)-(-)-Pregabalin filtriert wird.

13. Verfahren gemäss Anspruch 3 oder 12, **dadurch gekennzeichnet, dass** die erhaltene Mischung aus racemischem und (R)-(-)-Pregabalins mit Oxalsäure, die ein Moläquivalent der racemischen Menge ist, und Salzsäure, die ein Moläquivalent des Enantiomerprodukts ist, umgesetzt und dann das ausgefällte racemische Pregabalin (2) filtriert wird.

14. Verfahren gemäss Anspruch 1 oder 2, **dadurch gekennzeichnet, dass** Ammoniumacetat in der durch Spaltung mit (2R,3R)-(+)-Weinsäure erhaltenen Mutterlauge gelöst wird und dann Ammoniumhydrogentartrat und racemisches Pregabalin (2) ausgefällt werden.

## Revendications

1. Processus pour la préparation de (S)-(+)-prégabaline (1), **caractérisé en ce que** de la prégabaline racémique (2) est dissoute dans de l'eau,
est soit mise à réagir avec un acide α-hydroxy-carboxylique chiral en présence d'un acide achiral, soit mise à réagir avec un mélange d'acides α-hydroxy-carboxylique chiraux optionnellement en présence d'un acide achiral, dans lequel l'acide α-hydroxy-carboxylique chiral est l'acide (2R, 3R)-(+)-tartrique ou l'acide (S)-(+)-mandélique,
de la (S)-(+)-prégabaline (1) est libérée à partir du sel diastéréomère obtenu et optionnellement, le résidu de prégabaline racémique (2) est régénéré.

2. Processus selon la revendication 1, **caractérisé en ce que** de la prégabaline racémique (2) est mise à réagir en solution aqueuse avec 0,5-1,0 équivalent molaire d'un premier acide α-hydroxy-carboxylique chiral et 0,5-0,0 équivalent molaire d'un second acide α-hydroxy-carboxylique chiral en présence d'un acide achiral.

3. Processus selon la revendication 1, **caractérisé en ce que** de la prégabaline racémique (2) est mise à réagir avec 0,5-0,9 équivalent molaire d'un premier acide α-hydroxy-carboxylique chiral et 0,5-0,1 équivalent molaire d'un second acide α-hydroxy-carboxylique chiral en présence d'acide achiral en solution aqueuse.

4. Processus selon l'une quelconque des revendications 1 à 3, **caractérisé en ce que** le sel d'acide α-hydroxy-carboxylique de (S)-(+)-prégabaline (1) est filtré, cristallisé à partir de l'eau et mis à réagir par de l'hydroxyde d'ammonium aqueux, et le produit cristallin (S)-(+)-prégabaline (1) obtenu est filtré et recristallisé à partir de l'eau.

5. Processus selon l'une quelconque des revendications 1 à 4, **caractérisé en ce que** le sel d'acide α-hydroxy-carboxylique de (S)-(+)-prégabaline (1) est cristallisé pendant 1 heure, filtré et recristallisé à partir de l'eau.

6. Processus selon l'une quelconque des revendications 1 à 4, **caractérisé en ce que** le sel d'acide α-hydroxy-carboxylique chiral de (S)-(+)-prégabaline (1) est cristallisé pendant 12 heures, filtré et recristallisé deux fois à partir de l'eau.

7. Processus selon la revendication 5, **caractérisé en ce que** le liquide mère est cristallisé pendant 12 heures, filtré et le sel diastéréomère obtenu et le liquide mère sont réunis, de la prégabaline racémique (2) et de l'acide α-hydroxy-carboxylique chiral sont ajoutés puis le sel diastéréomère contenant la (S)-(+)-prégabaline (1) est cristallisé.

8. Processus selon l'une quelconque des revendications 1 à 7, **caractérisé en ce que** l'on utilise, en tant qu'acide achiral, de l'acide chlorhydrique, de l'acide benzoïque, de l'acide salicylique ou de l'acide phénylacétique.

9. Processus selon l'une quelconque des revendications 1 à 8, **caractérisé en ce que** le sel diastéréomère contenant de la (S)-(+)-prégabaline (1) recristallisée est dissous dans de l'hydroxyde d'ammonium aqueux chaud et la (S)-(+)-prégabaline précipitée (1) est filtrée.

10. Processus selon l'une quelconque des revendications 1 à 9, **caractérisé en ce que** la (S)-(+)-prégabaline (1) est recristallisée.

11. Processus selon la revendication 9, **caractérisé en ce que** le liquide mère obtenu est acidifié avec de l'acide chlorhydrique et l'acide S-(+)-mandélique précipité est filtré et, optionnellement, ce processus est répété.

12. Processus selon la revendication 4, **caractérisé en ce que** le premier liquide mère est mis à réagir avec de l'hydroxyde d'ammonium et le mélange précipité de racémique et de (R)-(-)-prégabaline est filtré.

13. Processus selon la revendication 3 ou 12, **caractérisé en ce que** le mélange obtenu de racémique et de (R)-(-)-prégabaline est mis à réagir avec de l'acide oxalique en quantité équimolaire du racémique et de l'acide chlorhydrique en quantité équimolaire du produit énantiomère, puis la prégabaline racémique précipitée (2) est filtrée.

14. Processus selon la revendication 1 ou 2, **caractérisé en ce que** de l'acétate d'ammonium est dissous dans le liquide mère obtenu par la résolution avec de l'acide (2R, 3R)-(+)-tartrique et puis de l'hydrogénotartrate d'ammonium et de la prégabaline racémique (2) sont précipités.
